(19) Europäisches Patentamt
European Patent Office
Office européen des brevets

(11) Publication number: **0 090 681**
**B1**

(12) # EUROPEAN PATENT SPECIFICATION

(45) Date of publication of patent specification: **30.12.86**

(21) Application number: **83400377.4**

(22) Date of filing: **23.02.83**

(51) Int. Cl.[4]: **C 07 D 471/04,**
C 07 D 213/75, A 61 K 31/495
// (C07D471/04, 241:00,
221:00)

(54) **Novel 1,2-dihydropyrido-(3,4-b)pyrazines.**

(30) Priority: **26.03.82 US 362480**

(43) Date of publication of application:
**05.10.83 Bulletin 83/40**

(45) Publication of the grant of the patent:
**30.12.86 Bulletin 86/52**

(84) Designated Contracting States:
**AT BE CH DE FR GB IT LI LU NL SE**

(56) References cited:
**JOURNAL OF MEDICINAL CHEMISTRY, vol. 17, no. 5, 1974, pages 552-553, Washington, US**
**JOURNAL OF ORGANIC CHEMISTRY, vol. 36, no. 19, 1971, pages 2818-2823, Washington, US**
**CHEMICAL ABSTRACTS, vol. 96, no. 11, March 15, 1982, page 34, no. 79528s, Columbus, Ohio, US**
**JOURNAL OF MEDICINAL CHEMISTRY, vol. 25, no. 9, September 1982, pages 1045-1050, Washington, US**
**J.A.C.S. 89(10), 1967, 2416-2423**

(73) Proprietor: **Southern Research Institute**
**2000 Ninth Avenue South**
**Birmingham Alabama 35205 (US)**

(72) Inventor: **Temple, Carroll G., Jr.**
**2224 Lynnchester Circle**
**Birmingham Alabama 35215 (US)**
Inventor: **Montgomery, John A.**
**3596 Springhill Road**
**Birmingham Alabama 35213 (US)**
Inventor: **Elliott, Robert D.**
**3517 Teton Circle**
**Birmingham Alabama 35216 (US)**
Inventor: **Wheeler, Glynn P.**
**2 East Wood Circle**
**Brimingham Alabama 35209 (US)**

(74) Representative: **Chameroy, Claude et al**
**c/o Cabinet Malemont 42, avenue du Président Wilson**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 090 681 B1

**Description**

Background of the Invention

This invention relates to novel 1,2-dihydropyrido[3,4-*b*]pyrazines, also known as 1-deaza-7,8-dihydropteridines. This invention also relates to a process for making such compounds and to novel intermediates obtained in said process.

The antimitotic chemical agents commonly known as spindle poisons are plant products of which the best known are colchicine, podophyllotoxin, and the vinca alkaloids. [L. Wilson, J. R. Bamburg, S. B. Mizel, L. M. Grisham and K. M. Creswell, *Federation Proceedings, 33,* 158 (1974)]. Two members of the latter, vincristine and vinblastine, are currently used clinically in the treatment of neoplasms. Although these agents produce a number of biochemical actions such as the inhibition of macromolecular synthesis, their primary effect is to prevent mitosis by interfering with the function of microtubules, which results in the accumulation of cells in metaphase. In addition, several benzimidazol-2-yl carbamates have been introduced as fungicides, anthelmintics and antitumoral agents. [L. C. Davidse and W. Flach *J. Cell Biol., 72,* 174 (1977)]. Those compounds also prevent mitosis and their biological activity can probably be attributed to interference with the formation or functioning of microtubules.

The development of procedures for the preparation of 1-deazapteridines is reported by J. A. Montgomery and N. F. Wood, *J. Org. Chem., 29,* 734 (1964); R. D. Elliot, C. Temple, Jr and J. A. Montgomery, *J. Org. Chem., 33,* 533 (1968); R. D. Elliot, C. Temple, Jr., J. L. Frye and J. A. Montgomery, *J. Org. Chem., 36* 2818 (1971); and R. D. Elliot, C. Temple, Jr. and J. A. Montgomery, *J. Med. Chem., 17,* 553 (1974). These references disclose the preparation and use of various 1,2-dihydro[3,4-*b*]pyrazine derivatives. Thus, the 1964 *J. Org. Chem.* reference discloses the compounds:

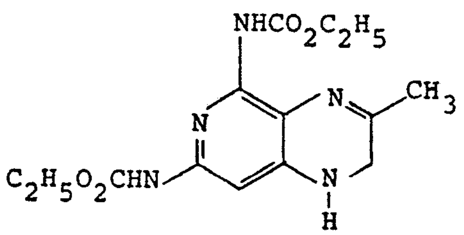

and

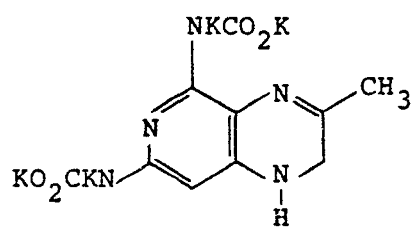

The 1968 *J. Org. Chem.* reference discloses the compound:

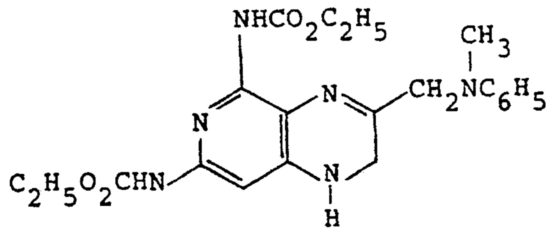

The 1971 *J. Org. Chem.* reference discloses the compounds:

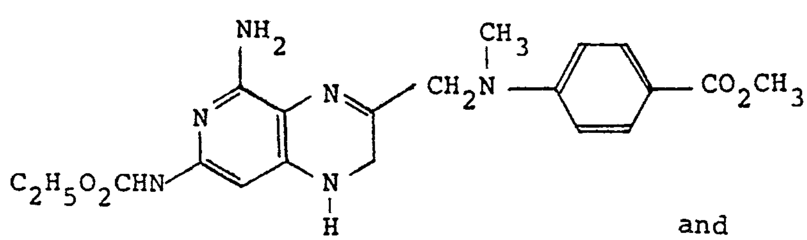

and

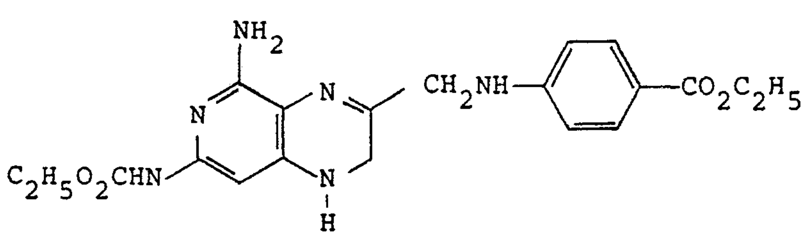

The *J. Med. Chem.* reference discloses that a dihydro-1-deazapteridine precursor of 1-deazamethotrexate showed activity against leukemia L1210 in mice. An abstract presented at the 28th Southeast Regional Meeting of the American Chemical Society in Gatlinburg, Tennessee, October 27—29, 1976 discloses that the compound

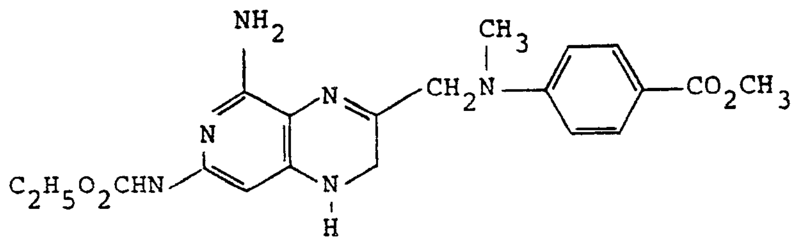

showed cytotoxicity in the KB cell culture screen and activity against leukemia L1210 in mice.

An abstract of a paper by B. J. Bowdon, G. P. Wheeler, C. G. Temple and J. A. Montgomery in AACR Abstracts, Vol. 22, March 1981 (page 25) discloses that a compound designated as "NSC—181928" was active against several neoplasms. NSC—181928 is the designation for the compound

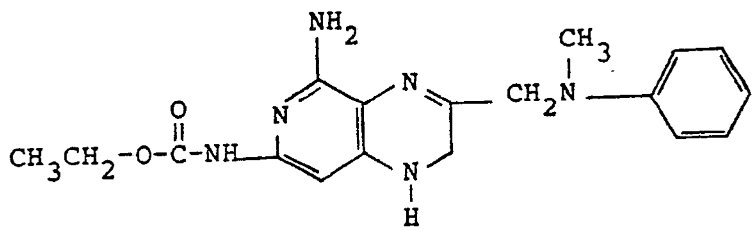

Summary of the Invention

It has now been found that certain 1,2-dihydropyrido[3,4-*b*]pyrazines which are not disclosed in any of the references discussed in the preceding section possess anticancer activity. The compounds of this invention have the structure:

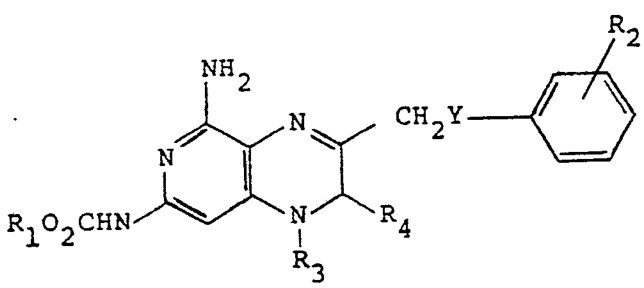

I

wherein the pair [$Y_1R_2$] is [$N(CH_3)$, 4-$OCH_3$], [$N(CH_3$, 4-Cl] or [$CH_2$, H]; and $R_3$ and $R_4$ are either both hydrogen or one is hydrogen and the other is a alkyl group containing up to 6 carbon atoms such as methyl, ethyl, propyl, butyl, etc. and $R_3$ and $R_4$ are either both hydrogen or one is hydrogen and the other is an alkyl group containing up to 6 carbon atoms.

Compounds of Formula I wherein $R_3$ is hydrogen may be prepared by aminating up to 6 carbon atoms, 3 containing alkyl ester of 6-amino-4-chloro-5-nitropyridin-2-ylcarbamate having the structure:

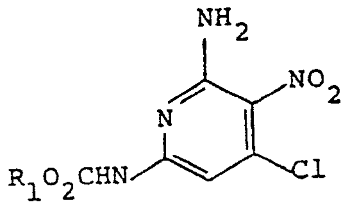

II

with the oxime of an alpha-amino ketone having the structure:

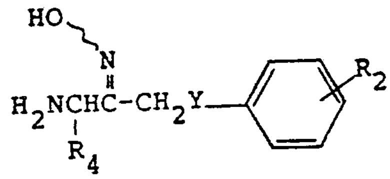

III

to give a compound having the structure:

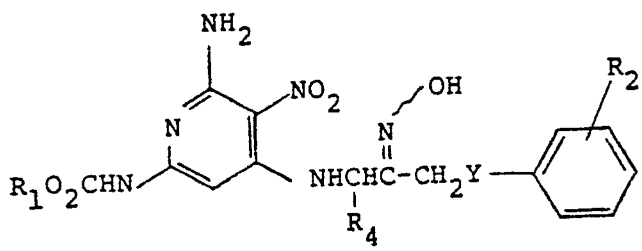

IV

wherein $R_1$, $R_2$, $R_4$ and Y are the same as previously defined. The compound of Formula IV is hydrolyzed, e.g., by acid hydrolysis to give the corresponding ketone having the formula:

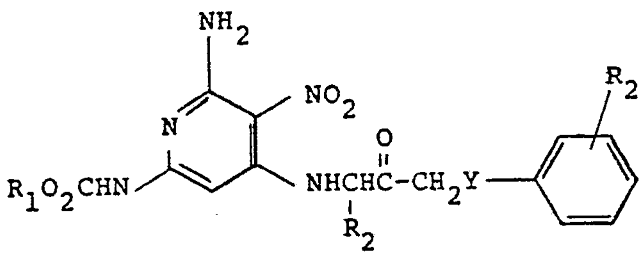

V

wherein $R_1$, $R_2$, $R_4$ and Y are the same as previously defined. The compound of Formula V is converted to the compound of Formula I by catalytic hydrogenation. An intermediate product formed during hydrogenation has the formula:

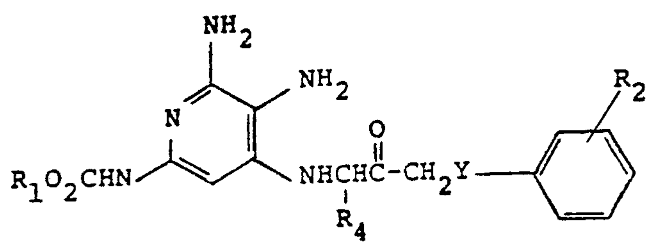

VI

wherein $R_1$, $R_2$, $R_4$ and Y are the same as previously defined.

Compounds of formula I wherein $R_3$ is hydrogen may be prepared by aminating an up to 6 carbon atoms containing alkyl ester of 6-amino-4-chlor-5-nitropyrimidin-2-ylcarbamate with the oxime of an alpha-amino ketone having the structure:

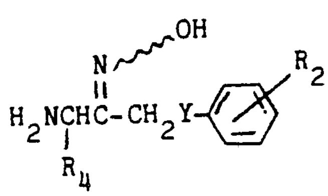

to obtain an up to 6 carbon atoms containing alkyl ester of a 6-amino-5-nitro-4[(2-oxoethyl)amino]pyridin-2-ylcarbamate oxime having the structure:

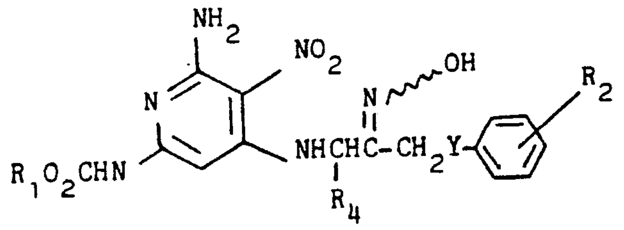

and subjecting said compound to catalytic hydrogenation, $R_1$, $R_2$, $R_4$ and Y being the same as previously defined.

Compounds of Formula I wherein $R_4$ is hydrogen may be prepared by aminating the compound of Formula II with an alpha-amino alcohol having the structure:

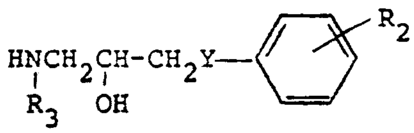

IIIA

to give a compound having the structure:

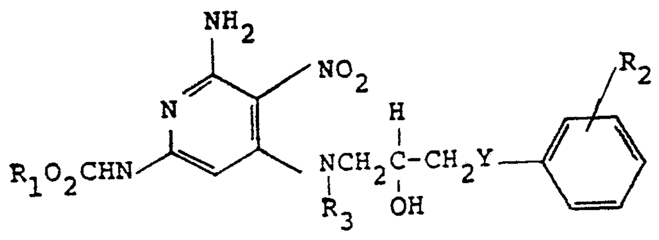

VII

which is oxidized to give a ketone having the structure:

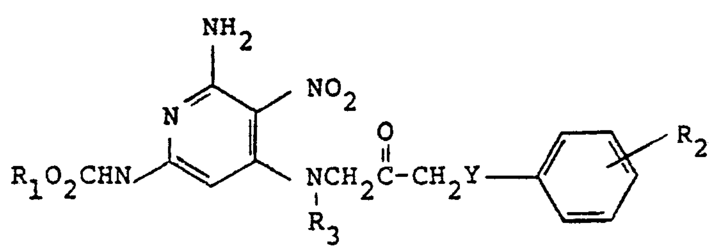

VA

wherein $R_1$, $R_2$, $R_3$ and Y are the same as previously defined. The compound of Formula VA is converted to a compound of Formula I by catalytic hydrogenation.

Detailed Description of the Invention

The compounds of the invention form pharmaceutically acceptable salts with both organic and inorganic acids. Examples of suitable acids for salt formation are hydrochloric, sulfuric, phosphoric, acetic, citric, oxalic, malonic, salicyclic, malic, fumaric, succinic, ascorbic, maleic, methanesulfonic, and the like. The salts are prepared by contacting the free base form with an equivalent amount of the desired acid in the conventional manner. The free base forms may be regenerated by treating the salt form with a base. For example, dilute aqueous base solution may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar

solvents, but the salts are otherwise equivalent to their respective free base forms for purposes of the invention.

Also embraced within the purview of the present invention are therapeutic compositions of matter useful for ameliorating cancer diseases in mammals and containing the 1-deaza-7,8-dihydropteridines of this invention or pharmaceutically acceptable salts thereof.

The active ingredients of the therapeutic compositions and the novel compounds of the present invention inhibit transplanted mouse tumor growth when administered in amounts ranging from about 5 mg to about 200 mg per kilogram of body weight per day. A preferred dosage regimen for optimum results would be from about 5 mg to about 50 mg per kilogram of body weight per day, and such dosage units are employed that a total of from about 350 mg to about 3.5 grams of the active compound for a subject of about 70 kg of body weight are administered in a 24-hour period. This dosage regimen may be adjusted to provide the optimum therapeutic response. For example, several divided doses may be administered daily or the dose may be proportionally reduced as indicated by the exigencies of the therapeutic situation. A decided practical advantage is that the active compound may be administered in any convenient manner such as by the oral, intravenous, intramuscular or subcutaneous routes.

The active compounds may be orally administered, for example, with an inert diluent or with an assimilable edible carrier, or they may be enclosed in hard or soft shell gelatin capsules, or they may be compressed into tablets, or they may be incorporated directly with food of the diet. For oral therapeutic administration, the active compounds may be incorporated with excipients and used in the form of ingestible tablets, buccal tablets, troches, capsules, elixirs, suspensions, syrups, wafers and the like. Such compositions and preparations should contain at least 0.1% of active compound. The percentage of the compositions and preparations may, of course, be varied and may conveniently be between about 2 and about 60% of the weight of the unit. The amount of active compound in such therapeutically useful compositions is such that a suitable dosage will be obtained. Preferred compositions or preparations according to the present invention are prepared so that an oral dosage unit form contains between 5 and about 200 milligrams of active compound.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or flavoring agent such as peppermint, oil of wintergreen or cherry flavoring. When the dosage unit form is a capsule, it may contain, in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compounds may be incorporated into sustained-release preparations and formulations.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene, glycol, and liquid polyethylene glycol, and the like), suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of the sterile powders for the preparations of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions indosage unit form for ease of administration and uniformity of dosage. Dosage unit form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically-acceptable carrier in dosage unit form as hereinbefore disclosed. A unit dosage form can, for example, contain the principal active compound in amounts ranging from about 0.1 to 400 mg, with from about one to about 30 mg being preferred. Expressed in proportions, the active compound is generally present in from about 0.1 to about 400 mg/ml of carrier. In the case of compositions containing supplementary active ingredients, the dosage are determined by reference to the usual dose and manner of administration of the said ingredients.

A preferred up to 6 carbon atoms containing alkyl ester of 6-amino-4-chloro-5-nitropyridin-2-ylcarbamate is the ethyl ester, i.e., ethyl 6-amino-4-chloro-5-nitropyridin-2-ylcarbamate. This compound is prepared according to the procedure described by R. D. Elliott, C. Temple, Jr. and J. A. Montgomery, *J. Org. Chem., 31,* 1890 (1966).

Oximes of alpha-amino ketones i.e., compounds of Formula III, may be prepared by known prior art procedures. Thus, they can be prepared by condensing the corresponding alpha-amino ketones with hydroxylamine hydrochloride in a refluxing mixture of pyridine and ethanol to give the oxime derivatives [R. D. Elliott, C. Temple, Jr. and J. A. Montgomery, *J. Org. Chem., 35,* 1674 (1970)].

The compounds of Formula III can also be prepared by alkylation of phthalimide with the corresponding alpha-bromo-ketone, treatment of the alpha-(phthalimido)-ketone product with hydroxylamine, and removal of the phthaloyl protecting group from the resulting oxime with hydrazine [R. D. Elliott, C. Temple, Jr. and J. A. Montgomery, *J. Org. Chem., 35,* 1676 (1970)].

Examples of these two procedures for the preparation of compounds of Formula III follow:

Method I

1-Amino-3-[[*N*-(4-methoxyphenyl)-*N*-methyl]amino]propane Oxime

A mixture of 1-bromo-3-(phthalimido)propanone (1.00 g., 3.54 mmol), N-methyl-p-anisidine (487 mg, 3.54 mmol), and $NaHCO_3$ (298 mg, 3.54 mmol) in DMAC (*N,N*-dimethyl acetamide) (10 ml) was stirred at room temperature for 20 hours, slowly diluted with $H_2O$ (3 ml) and stirred for 30 minutes. The yellow precipitate was collected by filtration, washed with DMAC—$H_2O$ (10:3) then $H_2O$ and dried *in vacuo* over $P_2O_5$: yield 927 mg. Dilution ofthe filtrate with additional $H_2O$ gave a second crop of product: yield 131 mg. Recrystallization of the combined crops from ethanol gave the diaminopropanone: yield 834 mg. Mass spectrum: m/e 338 $(M)^+$.

A solution of this product (600 mg, 1.77 mmol), hydroxylamine hydrochloride (184 mg, 2.65 mmol) and pyridine (2 ml) in ethanol (8 ml) was refluxed for 2 hours and evaporated to dryness *in vacuo*. The residue was washed with $H_2O$ (2 × 5 ml), then triturated with ethanol (2.5 ml) until a homogeneous yellow powder formed. The mixture was cooled in an ice bath and the oxime was collected by filtration, washed with cold ethanol and dried *in vacuo* over $P_2O_5$: yield 516 mg. Mass spectrum: m/e 353 $(M)^+$.

A solution of the oxime (400 mg, 1.13 mmol) in ethanol (15 ml) at 70°C was treated dropwise with a solution of 95% hydrazine (39.2 µl, 1.16 mmol) in ethanol (2 ml). The resulting solution was heated at 40°C for 17 hours, cooled to 25°C, treated with 1 N HCl (1.13 ml), stirred for 1 hour, cooled to 0°C and filtered to remove phthalhydrazide. The filtrate was evaporated to dryness *in vacuo* (< 40°C) and the residue was stirred with $H_2O$ (2 ml) and again evaporated to dryness *in vacuo*. An extract of the residue in $H_2O$ (5 ml) at 60°C was cooled to 25°C, filtered, cooled in an ice bath, treated with concentrated $NH_4OH$ (76 µl) and stirred at 0°C until a homogeneous tan solid formed. The alpha-aminoketone oxime was collected by filtration, washed with cold $H_2O$ and dried *in vacuo* over $P_2O_5$: yield 124 mg. Mass spectrum: m/e 223 $(M)^+$.

Method II

1-Amino-4-phenyl-2-butanone Oxime

A solution of crude 1-amino-4-phenyl-2-butanone hydrochloride (9.84 g, 49.2 mmol) (Degraw, J., Isakotellis, P., Kisliuk, R., and Gaumont, Y., *J. Heterocyclic Chem., 1971, 8,* 105), hydroxylamine hydrochloride (6.84 g, 98.4 mmol) and sodium acetate $3H_2O$ (13.4 g, 98.4 mmol) in 50% ethanol (250 ml) was heated at 75—80°C for 30 minutes, filtered, treated with a hot solution of picric acid (11.7 g, 51.1 mmol),

cooled to 25°C, filtered and allowed to stand for 2 days. The crystalline picrate was collected, washed with 2:1 water-ethanol and dried *in vacuo*: yield 9.62 g, mp, 151°C (Kofler Heizbank). The mothe liquor was evaporated to drynes *in vacuo,* and the residue was crystallized from hot water (500 ml) to give an additional amount of the picrate: yield 3.62 g, mp 151°C. A solution of the picrate in 3:1 ethanol-water (400 ml) was treated with washed BioRad AGI-X8 ($Cl^-$) ion exchange resin (100 g) and stirred for 18 hours. The solution was filtered and the resin was washed with 3:1 ethanol-water. The filtrate and wash were treated with additional resin (40 g), stirred for 2 hours and filtered. The almost colorless solution was evaporated with ethanol (3 × 200 ml). The residue was stirred with ethanol (100 ml), filtered and the precipitate was rinsed with additional ethanol (40 ml). The filtrate and wash were diluted with diethyl ether (600 ml) to give a crystalline hydrochloride which was collected, washed with diethyl ether and dried *in vacuo* ($P_2O_5$): yield, 5.36 g.

Alpha-amino alchohols of Formula IIIA are prepared by the following procedure:

Method III

1-Amino-3-[[*N*-(4-chlorophenyl)-N-methyl]amino]2-propanol

A solution of epichlorohydrin (11 ml) and 4-chloro-*N*-methylaniline (11 g, 78 mmol) in a mixture of ethanol (10 ml) and water (7 ml) was refluxed for 2 hours, diluted with water (20 ml), and extracted with diethyl ether (3 × 50 ml). The combined extracts were evaporated to dryness, the residue was treated with a solution of NaOH (5 g) in water (10 ml) for 1 hour, and the resulting mixture was extracted with diethyl ether (4 × 25 ml). The combined extracts were dried ($MgSO_4$) and evaporated to dryness *in vacuo* to give 1-[[*N*-(4-chlorophenyl)-*N*-methyl]amino]-2,3-epoxypropane: yield, 11 g (72%). A solution of this sample in a mixture of ethanol (50 ml) and liquid $NH_3$ (20 ml) was heated in a glass-lined stainless steel bomb at 100°C for 3 hours. The resulting reaction solution was evaporated to dryness, and the dried residue was recrystallized from $C_6H_5$: yield, 5.3 g (44%).

The oximes and alpha-amino alcohols set forth in Table I were prepared by Method I, Method II or Method III, as indicated in Table I. The first column of Table I sets forth the structure of the group

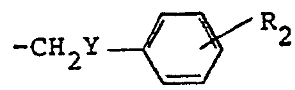

in Formula III for the oximes prepared by Methods I and II and in Formula IIIA for the alpha-amino alcohol prepared by Method III.

TABLE I

Alpha-Aminoketone Oximes (III) and Alpha-Aminoalcohols (IIIA)

| Compound[a] | Method | Yield %[b] | M.p.°C | Formula | Analysis Calcd, % C | Analysis Calcd, % H | Analysis Calcd, % N | Analysis Found, % C | Analysis Found, % H | Analysis Found, % N |
|---|---|---|---|---|---|---|---|---|---|---|
| 4—$CH_3OC_6H_4N(CH_3)CH_2$ | I | 28 | [c] | $C_{11}H_{17}N_3O\cdot 0.37H_2O$ | 57.46 | 7.78 | 18.27 | 57.39 | 7.70 | 18.38 |
| $C_6H_5CH_2CH_2$ | II | 50 | 193 | $C_{10}H_{14}N_2O\cdot HCl\cdot 0.3H_2O$ | 54.82 | 6.72 | 12.79 | 54.63 | 7.00 | 12.58 |
| 4—$ClC_6H_4N(CH_3)CH_2$ | III | 32 | 117—9[d] | $C_{10}H_{15}ClN_2O$ | 55.95 | 7.04 | 13.05 | 56.29 | 7.18 | 13.17 |

[a] $R_3$ and $R_4$ = H

[b] Overall yield

[c] Indefinite melting point

[d] Resolidified and remelted at 123°C

A compound of Formula II is aminated with a compound of Formula III under nitrogen in refluxing ethanol containing triethylamine as an acid acceptor to give a compound of Formula IV. An example of this procedure follows:

Example 1

Ethyl 6-Amino-4-[3-[[*N*-(4-methoxyphenyl)-*N*-methyl]amino]-2-oxopropylamino]-5-nitro-2-pyridinecarbamate Oxime

(IV: $R_1 = C_2H_5$; $R_2$ = 4—$CH_3O$; $R_4$ = H; Y = $N(CH_3)$)

A solution of ethyl 6-amino-4-chloro-5-nitro-2-pyridinecarbamate (5.00 g, 19.2 mmol), 1-amino-3-[[*N*-(4-methoxyphenyl)-*N*-methyl]amino]propanone oxime (4.29 g, 19.2 mmol) and triethylamino (1.94 g, 19.2 mmol) in $CH_3OH$ (75 ml) was refluxed under $N_2$ for 15 hours and cooled to 25°C. The yellow crystalline precipitate was collected by filtration, washed with ethanol and dried *in vacuo.* A solution of the crude product (6.46 g) in DMAC (260 ml) was diluted with $H_2O$ (430 ml) to give a precipitate which was collected by filtration, washed with $H_2O$ and dried *in vacuo* over $P_2O_5$: yield 6.03 g. Mass spectrum: m/e 447 $(M)^+$.

Additional compounds were prepared similarly wherein the oxime of Example I was replaced with other oximes. The properties of these compounds are set forth in Table II. The first column of Table II sets forth the structure of the group:

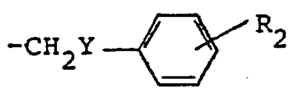

in starting alpha-aminoacetophenone oxime, see Formula III wherein $R_1$ is $C_2H_5$ and $R_4$ is H, and in the final product, see Formula IV, wherein $R_1H_5$ and $R_4$ is H.

TABLE II

Ethyl 4-(Substituted)amino-6-amino-5-nitro-pyridine-2-carbamate Oximes

| Compound | Reaction Time, Hours | Yield % | M.p. °C | Formula | Analyses Calcd, % C | H | N | Found, % C | H | N |
|---|---|---|---|---|---|---|---|---|---|---|
| 4—$CH_3OC_6H_4N(CH_3)CH_2$ | 15[a] | 75 | 190 | $C_{19}H_{25}N_7O_6$ | 51.00 | 5.63 | 21.91 | 50.97 | 5.89 | 21.87 |
| $C_6H_5CH_2CH_2$ | 24[b] | 84 | 187 | $C_{18}H_{22}N_6O_5$ | 53.72 | 5.51 | 20.89 | 53.61 | 5.56 | 20.77 |

[a]Solvent, methanol
[b]Reaction temperature 54°C

Treatment of a compound of Formula IV with a 1:1 mixture of 1 *N* hydrochloric acid and dioxane at 60°C hydrolyzes the oxime function to give a compound of Formula V. An example of this procedure follows:

Example 2

Ethyl 6-Amino-4-[2-oxo-4-(phenylbutyl)-amino]-5-nitro-2-pyridinecarbamate
(V: $R_1 = C_2H_5$; $R_2 = H$; $R_4 = H$; $Y = CH_2$)

A solution of the oxime of the title compound (6.17 g, 15.4 mmol) in warm dioxane (60 ml) was treated with 1 *N* HCl (120 ml), stirred at 55°C for 1 hour and cooled in an ice bath. The precipitated hydrochloride was collected, washed with cold water, then suspended in water (300 ml) and neutralized with 1 *N* NaOH. The yellow product was collected, washed with water and dried *in vacuo* ($P_2O_5$): yield, 5.08 g (83%); m.p. 155°C. Anal. Calcd. for $C_{18}H_{22}N_5O_5$: C, 55.80; H, 5.46; N, 18.08. Found: C, 55.42; H 5.49; N, 18.12.

Amination of the compound of Formula II with the compound of Formula IIIA in refluxing ethanol containing triethylamine as an acid acceptor gives a compound of Formula VII. Oxidation of a compound of formula VII gives a ketone of Formula VA. Two examples of this procedure follow:

Example 3

A. Ethyl 6-Amino-4-[3-[[*N*-(4-chlorophenyl)-*N*-methyl]amino]-2-hydroxypropyl-amino]-5-nitro-2-pyridinecarbamate
(VII: $R_1 = C_2H_5$; $R_2 = 4{-}Cl$; $R_3 = H$; $Y = N(CH_3)$)

A solution of ethyl 6-amino-4-chloro-5-nitro-2-pyridinecarbamate (10.0 g), 1-amino-3-[[*N*-(4-chlorophenyl)-*N*-methyl]amino]-2-propanol (8.25 g), and triethylamine (10.7 ml) in methanol (120 ml) was heated 60°C for 18 hours and evaporated to dryness *in vacuo*. The dark residue was washed with diethyl ether (1.5 l) to give a yellow solid. This solid was washed with water (50 ml) and recrystallized twice from a mixture of ethanol and hexane: yield, 4.92 g. Similar treatment of the residue obtained from the ether wash from above gave a slightly impure sample of product: yield, 5.89 g. Total yield, 10.81 g (64%); m.p., 181°C. Anal. Calcd. for $C_{18}H_{23}ClN_6O_5$: C, 49.26; H, 5.28; N, 19.15. Found: C, 49.16; H, 5.46; N, 19.22.

B. Ethyl 6-Amino-4-[3-[[*N*-(4-chlorophenyl)-*N*-methyl]amino]-2-oxoropyl-amino]-5-nitro-2-pyridinecarbamate
(VA: $R_1 = C_2H_5$; $R_2 = 4{-}Cl$; $R_3 = H$; $Y = N(CH_3)$)

A solution of ethyl 6-amino-4-[3-[[*N*-(4-chlorophenyl)-*N*-methyl]amino]-2-hydroxypropylamino]-5-nitro-6-pyridinecarbamate (1.76 g), and acetic anhydride (8 ml) in dimethyl sulfoxide (40 ml) was stirred at room temperature for 20 hours diluted with water (200 ml), and neutralized to pH 5.2 with 1 *N* NaOH. The solid that deposited was collected by filtration and dissolved in $CHCl_3$. Evaporation of this solution to dryness and trituration of the resulting solid successively with water and ethanol gave the product yield, 0.36 g (20%); m.p. 123—5°C. Anal. Cald. for $C_{18}H_{21}ClN_6O$: C, 48.49; H, 4.97; N, 18.85. Found: C, 48.74; H, 4.65; N, 18.89.

The catalytic hydrogenation of a compound of Formula V or VA with a three-fold amount of Raney nickel in a large volume of ethanol (i.e., more than one liter per gram) at atmospheric pressure at room temperature or with intermittent warming (e.g., to no higher than 60°C.) with a water bath gives the intermediate compound of Formula VI which is cyclized *in vacuo* with the elimination of water to give a compound of Formula I. Such reaction is shown in Example 4. The compounds of Formula I can also be prepared directly by hydrogenation of a compound of Formula IV in the presence of Raney nickel as shown in Example 5.

Example 4

Ethyl 5-Amino-1,2-dihydro-3-(2-phenylethyl)-pyrido(3,4-*b*)pyrazin-7-carbamate
(I: $R_1 = C_2H_5$; $R_2 = H$; $R_3 = H$; $Y = N(CH_2)$

A solution of ethyl 6-amino-4-[2-oxo-4-(phenylbutuyl)amino]-5-nitro-2-pyridinecarbamate (300 mg, 0.775 mmol) in *N,N'*-dimethyl acetamide (7 ml) was hydrogenated in the presence of Raney nickel (890 mg, weighed wet, washed with ethanol) for 20 hours to give an $H_2$ uptake of 58 ml (3.07 mmol). The reaction mixture was filtered under $N_2$ and evaporated *in vacuo* at 25°C. The residual syrup was stirred with water (10 ml) to give a white powder which was collected, washed with water and dried *in vacuo* ($P_2O_5$): yield, 230 mg. The properties are set forth in Table III.

Example 5

Ethyl 5-Amino-1,2-dihydro-3-[[*N*-(4-methoxyphenyl)-*N*-methyl]aminomethyl]pyrido-[3,4-*b*]pyrazine-7-carbamate
(I: $R_1 = C_2H_5$; $R_2 = 4{-}CH_3O$; $R_3 = H$; $R_4 = H$; $Y = N(CH_2)$)

A suspension of the oxime of ethyl 6-amino-4-[3-[[4-(*N*-(4-methoxyphenyl)-*N*-methyl]amino]-2-oxopropylamino]-5-nitro-2-pyridinecarbamate (672 mg, 1.50 mmol) and Raney nickel (2 g, weighed wet, washed with ethanol) in ethanol (90 ml) was hydrogenated at room temperature and atmospheric pressure with vigorous stirring. The hydrogen uptake (124 ml, 5.66 mmol) was complete in 2.75 hours. The mixture was filtered under $N_2$ and the catalyst extracted with boiling ethanol (3 × 30 ml). The combined filtrate and

extract were concentrated *in vacuo* to about 7 ml and cooled in an ice bath to deposit the product as a pale yellow crystalline solid: yield 421 mg. Mass spectrum: m/e 384 $(M)^+$. The properties are set forth in Table III.

The compounds set forth in Table III were prepared by the procedure of Example 5 and Example 6, as indicated in Table III. The properties of these compounds are set forth in Table III. The first column of Table III sets forth the structure of the group:

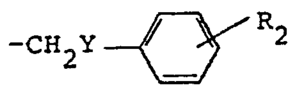

in the starting material, see Formula IV, V and VA wherein $R_2$ is $C_2H_5$ and $R_3$ and $R_4$ are hydrogen, and in the final product, see Formula I wherein $R_1$ is $C_2H_5$ and $R_3$ and $R_4$ are hydrogen.

TABLE III

Ethyl 3-Substituted 5-Amino-1,2-dihydropyrido-[3,4-*b*]pyrazine-7-carbamates

| Compound | Procedure of Example | Reaction Time, Hours | Yield, % | M.p.°C | Formula | Analyses Calcd, % C | H | N | Found, % C | H | N |
|---|---|---|---|---|---|---|---|---|---|---|---|
| $4-ClC_6H_4N(CH_3)CH_2$ | 4 | 20 | 16 | [a] | $C_{18}H_{21}ClN_6O_2 \cdot 0.5\ H_2O \cdot 0.5C_2H_6O$ | 54.22 | 5.99 | 19.97 | 54.06 | 5.96 | 19.83 |
| $4-CH_3OC_6H_4N(CH_3)CH_2$ | 5 | 3 | 73 | 187 | $C_{19}H_{24}N_6O_3$ | 59.36 | 6.29 | 21.86 | 59.16 | 6.29 | 20.47 |
| $C_6H_5CH_2CH_2$ | 4 | 20 | 88 | 163 | $C_{18}H_{21}N_5O_2$ | 63.70 | 6.24 | 20.64 | 63.35 | 6.25 | 20.47 |

[a]indefinite

The 1,2-dihydropyrido[3,4-*b*]pyrazines of this invention are powerful inhibitors of the proliferation of cultured lymphoid leukemia L1210 cells shown in Table IV. The concentration causing a 50% inhibition of proliferation of the cells during 24 hours is similar to that observed for vincristine, vinblastine, and colchicine. Also, the addition to the test medium of inosine, thymidine, glycine, citrovorum factor, individually and in combinations, and elevated concentration of amino acids and vitamins did not overcome the inhibitions.

In addition to cell cytotoxicity, the 1,2-dihydropyrido[3,4-*b*]pyrazines showed activityu against lymphocytic leukemia P388 cells ($10^6$) implanted intraperitoneally in mice. Ethyl 5-amino-1,2-dihydro-3-[(*N*methyl-*N*-phenyl)aminomethyl]pyrido[3,4-*b*]pyrazine-7-carbamate and ethyl 5-amino-1,2-dihydro-3-[[3,4-*b*]pyrazine-7-carbamate are also active in mice against P388 cells that were resistant to vincristine.

The 1,2-dihydropyrido[3,4-*b*]pyrazines of this invention at concentrations that prevented any increase in the cell number during a 24 hour period had little effect upon the synthesis of DNA, RNA, and protein by cultured L1210 cells during exposure for four hours. This result and those described above led to the determination of the effect of the 1,2-dihydropyrido[3,4-*b*]-pyrazines upon cell division. Exposure of cultured L1210 cells to the 1,2-dihydropyrido[3,4-*b*]pyrazines inhibited cell division as measured by the mitotic index (MI) (Table IV), which is the fraction of the cell population that is made up of metaphase cells. Sunbsequent experiments showed that these agents caused the accumulation in metaphase of human epidermoid carcinoma #2 cells, P388 cells, and P388 cells resistant to vincristine grown in suspension culture and of colon tumor #26n cells and colon tumor #38 cells grown on plastic surfaces.

Table IV sets forth biological data for 1-deaza-7,8-dihydropteridines of this invention and for three prior art compounds. The first column of Table IV set forth the structure of the group R in the formula in the heading of the table for the 1-deaza-7,8-dihydropteridines tested.

The data in Table IV shows that the 1-deaza-7,8-dihydropteridines of this invention are active against leukemia in laboratory animals.

TABLE IV
Biological Data — 1-Deaza-7,8-dihydropteridines

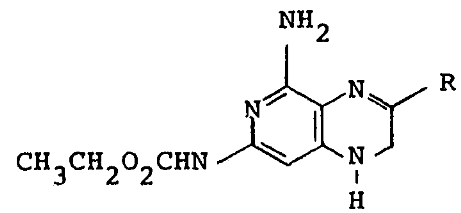

| Compound | L1210(a) $ID_{50}\mu M$ | Mitotic Index (b) 12h (μM) | Mitotic Index (b) 24h (μM) | P388 (c) $10^6$ Tumor cell implant, i.p. Schedule, Days | P388 (c) $10^6$ Tumor cell implant, i.p. %ILS (mg/kg) |
|---|---|---|---|---|---|
| Nocodazole | $27 \times 10^{-3}$ | | 0.19 (0.3) | | |
| Vincristine | $1 \times 10^{-3}$ | | 0.62 (0.3) | 1 | 100 (2.7) |
| 4—$CH_3OC_6H_4N(CH_3)CH_2$ | $7.9 \times 10^{-3}$(d) | 0.64 (0.003)<br>0.49 (0.3) | | 1<br>1 (f)<br>1 (g) | 80 (50) (e)<br>133 (25)<br>150 (25) |
| 4—$ClC_6H_4N(CH_3)CH_2$ | $14.5 \times 10^{-3}$ | 0.71 (0.03) | | 1 | 65 (100) |
| $C_6H_5CH_2CH_2$ | $13 \times 10^{-3}$ | 0.33 (0.3) | | 1 | 29 (100) |
| 4—$CH_3O_2CC_6H_4N(CH_3)CH_2$(h) | $58 \times 10^{-3}$ | 0.61 (0.3)<br>0.44 (0.1) | | 1—9 | 30(25) |

(a) Concentration of agent that inhibits proliferation of cultured lymphoid leukemia L1210 cells to 50% control growth during 48 hours. G. P. Wheeler, B. J. Bowdon, J. A. Werline, D. J. Adamson and C. Temple, Jr., *Cancer Res., 42,* 791 (1982).
(b) Fraction of the cell population of cultured lymphoid leukemia L1210 cells in mitosis (ref in a).
(c) Lymphocytic leukemia P388 R. I. Geran, N. H. Greenbert, M. M. MacDonald, A. M. Schumacker, and B. J. Abbott, *Cancer Chemother. Rep., 3* (2) (1972).
(d) Average of 2-determinations.
(e) 2-cures.
(f) Methotrexate-resistant line of P388 (designated tumor P7 by the Drug Evaluation Branch, National Cancer Institute). In mice with $10^6$ cell implant (IP), methothrexate at a dose of 2 mg/kg on the qd 1—9 schedule gave a 2-log cell kill against the sensitive line of P388 and a 2-log increase in cells against the methotrexate-resistant line of P388.
(g) Vincristine-resistant line of P388. L. J. Wilkoff and E. A. Dulmadge, *J. Natl. Cancer Inst., 61,* 1521 (1978).
(h) R. D. Elliott, C. Temple, Jr., J. L. Frye, and J. A. Montgomery, *J. Org. Chem., 36,* 2818 (1971).

**Claims for the Contracting States: BE CH DE FR GB IT LI LU NL SE**

1. A 1,2-dihydropyrido[3,4-*b*]pyrazine having the formula:

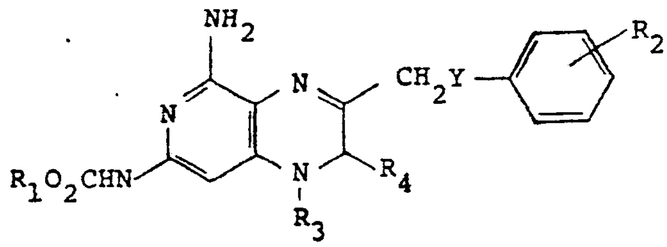

wherein the pair [$Y,R_2$] is [$N(CH_3)$, 4—$OCH_3$], [$N(CH_3)$, 4—Cl] or [$CH_2$, H];

2. A compound as defined in claim 1 wherein $R_1$ is ethyl.

3. A compound as defined in claim 2 wherein $R_3$ and $R_4$ are each hydrogen.

4. Ethyl 5-amino-1,2-dihydro-3-[[*N*-(4-methoxyphenyl)-*N*-methyl]aminomethyl]pyrido[3,4-*b*]pyrazine-7-carbamate.

5. Ethyl 5-amino-1,2-dihdyro-3-[[*N*-(4-chlorophenyl)-*N*-methyl]aminomethyl]pyrido[3,4-*b*]pyrazine-7-carbamate.

6. Ethyl 5-amino-1,2-dihydro-3-(2-phenylethyl) pyrido[3,4-*b*]pyrazine-7-carbamate.

7. A compound having the formula:

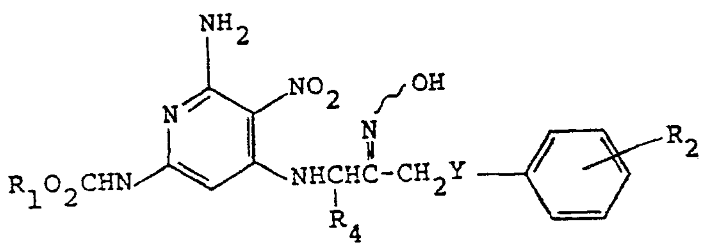

wherein the pair [$Y,R_2$] is [$N(CH_3)$, 4—$OCH_3$], [$N(CH_3)$, 4—Cl] or [$CH_2$, H];
$R_1$ is an alkyl group containing up to 6 carbon atoms; and
$R_4$ is hydrogen or an alkyl group containing up to 6 carbon atoms.

8. A compound as defined in claim 7 wherein $R_1$ is ethyl.

9. A compound as defined in claim 8 wherein $R_4$ is hydrogen.

10. A compound having the formula:

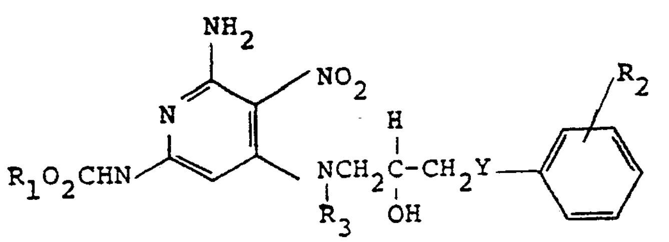

wherein the pair [$Y,R_2$] is [$N(CH_3)$, 4—$OCH_3$], [$N(CH_3)$, 4—Cl] or [$CH_2$, H];
$R_1$ is an alkyl group containing up to 6 carbon atoms; and $R_3$ is hydrogen or an alkyl group containing up to 6 carbon atoms.

11. A compound as defined in claim 10 wherein $R_1$ is ethyl.

12. A compound as defined in claim 11 wherein $R_3$ is hydrogen.

13. A compound having the formula:

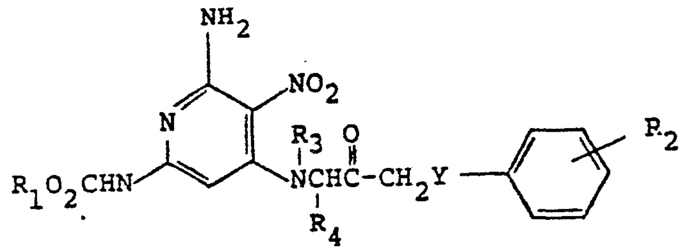

wherein the pair $[Y,R_2]$ is $[N(CH_3), 4—OCH_3]$, $[N(CH_3), 4—Cl]$ or $[CH_2, H]$; $R_1$ is an alkyl group containing up to 6 carbon atoms; and $R_3$ and $R_4$ are either both hydrogen or one is hydrogen and the other is an alkyl group containing up to 6 carbon atoms.

14. A compound as defined in claim 13 wherein $R_1$ is ethyl.

15. A compound as defined in claim 14 wherein $R_3$ and $R_4$ are each hydrogen.

16. A process for preparing a 1,2-dihydropyrido[3,4-*b*]pyrazine as defined in claim 1 which comprises aminating an up to 6 carbon atoms containing alkyl ester of 6-amino-4-chloro-5-nitropyrimidin-2-yl-carbamate with the oxime of an alpha-amino ketone having the structure:

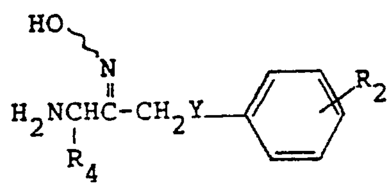

III

to obtain an up to 6 carbon atoms containing alkyl ester of a 6-amino-5-nitro-4-[(2-oxoethyl)amino]pyridin-2-ylcarbamate oxime having the structure.

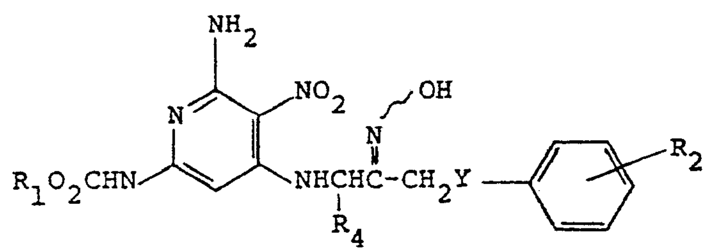

hydrolyzing said compound to obtain the corresponding ketone having the structure:

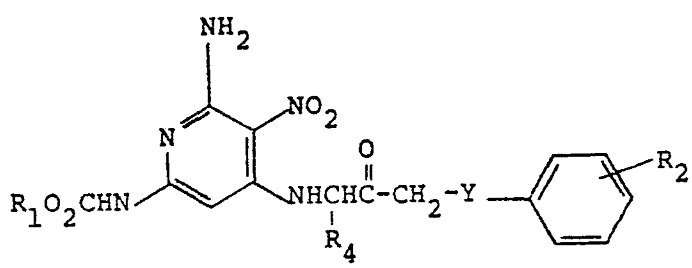

and subjecting said ketone to catalytic hydrogenation, wherein $R_1$, $R_2$, $R_4$ and Y are as defined in claim 1.

17. A process for preparing a 1,2-dihydropyrido[3,4-*b*]pyrazine as defined in claim 1 which comprises aminating an up to 6 carbon atoms containing alkyl ester of 6-amino-4-chloro-5-nitropyrimidin-2-ylcarbamate with the oxime of an alpha-amino ketone having the structure:

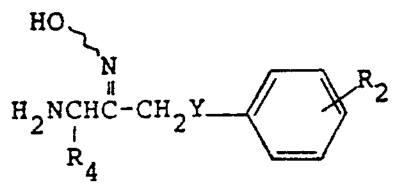

III

to obtain an up to 6 carbon atoms containing alkyl ester of a 6-amino-5-nitro-4-[(2-oxoethyl)amino]pyridin-2-ylcarbamate oxime having the structure.

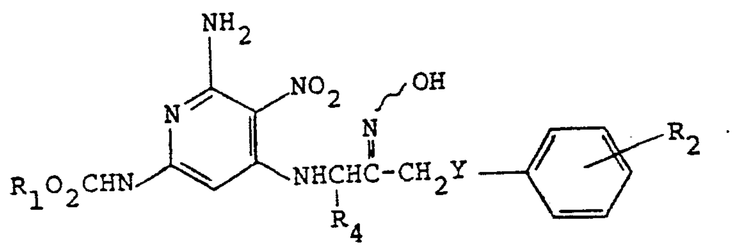

and subjecting said compound to catalytic hydrogenation, wherein $R_1$, $R_2$, $R_4$ and Y are as defined in claim 1.

18. A process for preparing a 1,2-dihydropyrido[3,4-*b*]pyrazine as defined in claim 1 which comprises aminating an up to 6 carbon atoms containing alkyl ester of 6-amino-4-chloro-5-nitropyridin-2-ylcarbamate with an alpha-amino alcohol having the structure:

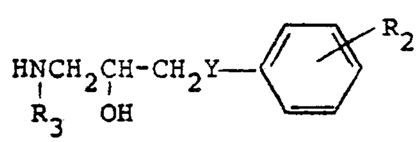

to give a compound having the structure:

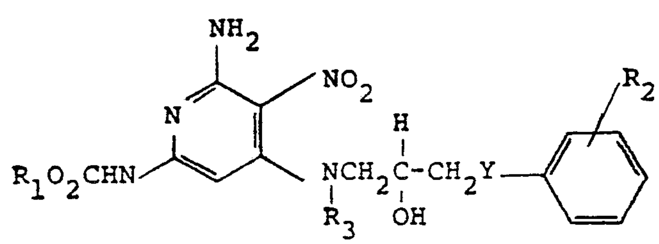

oxidizing said compound to give a ketone having the structure:

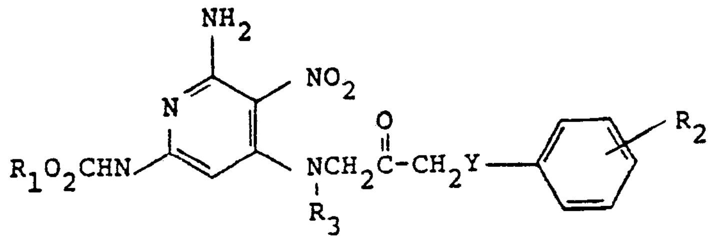

and subjecting said ketone to catalytic hydrogenation, wherein $R_1$, $R_2$, $R_3$ and Y are as defined in claim 1.

19. A pharmaceutical composition in dosage unit form comprising an effective amount of a compound as defined by claim 1 in association with a pharmaceutical carrier.

**Claims for the Contracting State: AT**

1. A process for preparing a 1,2-dihydropyrido[3,4-*b*]pyrazine having the formula:

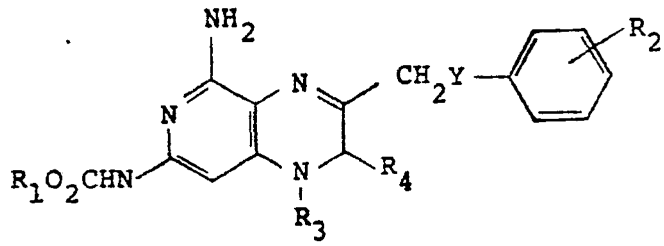

wherein the pair [Y,$R_2$] is [N($CH_3$), 4—$OCH_3$], [N($CH_3$), 4—Cl] or [$CH_2$, H]; which comprises aminating an up to 6 carbon atoms containing alkyl ester of 6-amino-4-chloro-5-nitro-pyrimidin-2-ylcarbamate with the oxime of an alpha-amino ketone having the structure:

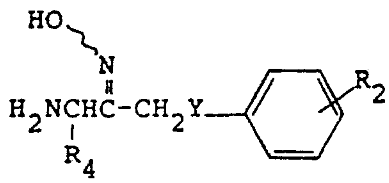

to obtain an up to 6 carbon atoms containing alkyl ester of a 6-amino-5-nitro-4-[(2-oxoethyl)amino]pyridin-2-ylcarbamate oxime having the structure;

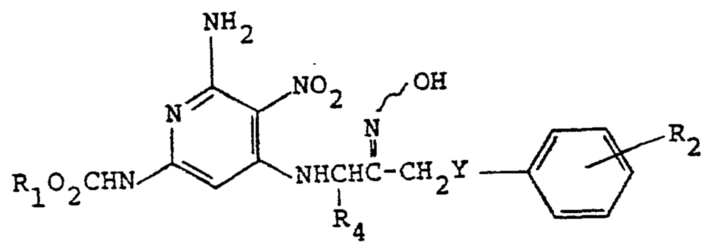

hydrolyzing said compound to obtain the corresponding ketone having the structure:

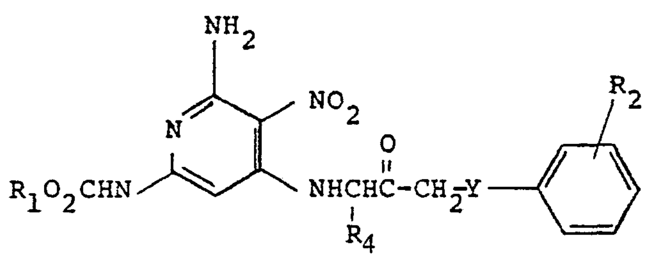

and subjecting said ketone to catalytic hydrogenation, wherein $R_1$, $R_2$, $R_4$ and Y are as defined above.

2. A process for preparing a 1,2-dihydropyrido[3,4-*b*]pyrazine as defined in claim 1 which comprises aminating an up to 6 carbon atoms containing alkyl ester of 6-amino-4-chloro-5-nitropyrimidin-2-yl-carbamate with the oxime of an alpha-amino ketone having the structure:

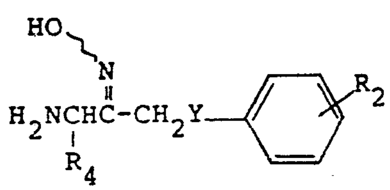

III

to obtain an up to 6 carbon atoms containing alkyl ester of a 6-amino-5-nitro-4-[(2-oxoethyl)amino]pyridin-2-ylcarbamate oxime having the structure:

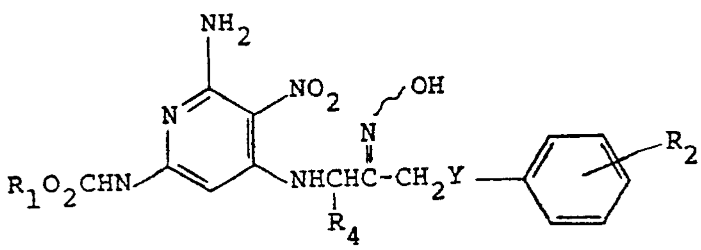

and subjecting said compound to catalytic hydrogenation, wherein $R_1$, $R_2$, $R_4$ and Y are as defined in claim 1.

3. A process for preparing a 1,2-dihydropyrido[3,4-*b*]pyrazine as defined in claim 1 which comprises aminating an up to 6 carbon atoms containing alkyl ester of 6-amino-4-chloro-5-nitropyridin-2-ylcarbamate with an alpha-amino alcohol having the structure:

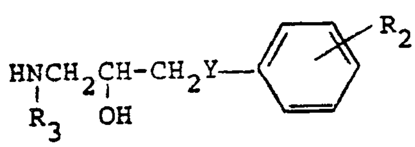

to give a compound having the structure:

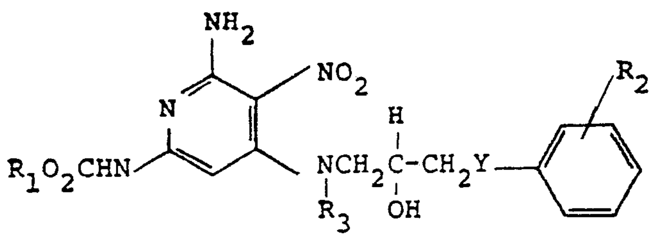

oxidizing said compound to give a ketone having the structure:

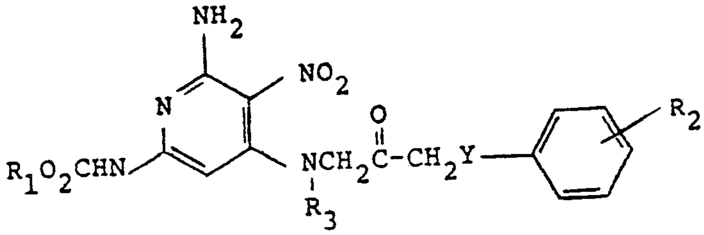

and subjecting said ketone to catalytic hydrogenation, wherein $R_1$, $R_2$, $R_3$ and Y are as defined in claim 1.

**Patentansprüche für die Vertragsstaaten: BE CH DE FR GB IT LI LU NL SE**

1. 1,2-Dihydropyrido[3,4-b]pyrazin der Formel:

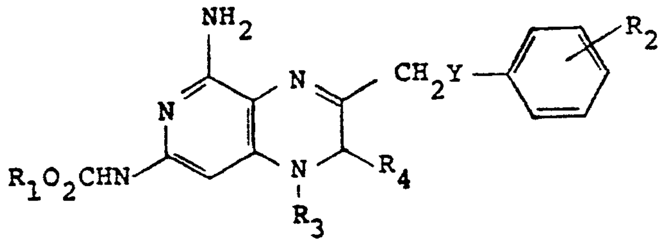

worin
das Paar [Y, $R_2$] die Bedeutung [$N(CH_3)$, 4—$OCH_3$], [$N(CH_3)$, 4—Cl] oder [$CH_2$, H] hat;
$R_1$ eine Alkylgruppe, die bis zu 6 Kohlenstoffatome enthält, bedeutet; und
$R_3$ und $R_4$ entweder beide Wasserstoff bedeuten oder eines Wasserstoff und das andere eine Alkylgruppe, die bis zu 6 Kohlenstoffatome enthält, bedeutet;
und pharmazeutisch annehmbare Salz hiervon.

2. Verbindung nach Anspruch 1, wobei $R_1$ Ethyl bedeutet.

3. Verbindung nach Anspruch 2, wobei $R_3$ und $R_4$ jeweils Wasserstoff bedeuten.

4. Ethyl-5-amino-1,2-dihydro-3-[[N-(4-methoxyphenyl)-N-methyl]aminoethyl]pyrido[3,4-b]pyrazin-7-carbamat.

5. Ethyl-5-amino-1,2-dihydro-3-[[N-(4-chlorphenyl)-N-methyl]-aminomethyl]pyrido[3,4-b]pyrazin-7-carbamat.

6. Ethyl-5-amino-1,2-dihydro-3-(2-phenylethyl)pyrido[3,4-b]pyrazin-7-carbamat.

7. Verbindung der Formel:

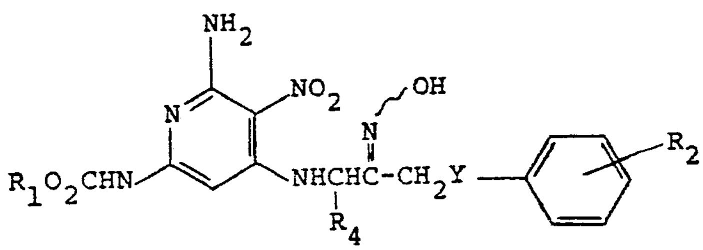

worin
das Paar [Y, $R_2$] die Bedeutung [$N(CH_3)$, 4—$OCH_3$], [$N(CH_3)$, 4—Cl] oder [$CH_2$, H] hat;
$R_1$ eine Alkylgruppe, die bis zu 6 Kohlenstoffatome enthält, bedeutet; und
$R_4$ Wasserstoff oder eine Alkylgruppe, die bis zu 6 Kohlenstoffatome enthält, bedeutet.

8. Verbindung nach Anspruch 7, wobei $R_1$ Ethyl bedeutet.

9. Verbindung nach Anspruch 8, wobei $R_4$ Wasserstoff bedeutet.

10. Verbindung der Formel:

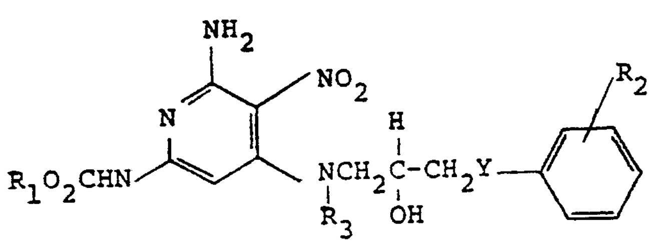

worin
das Paar [Y, $R_2$] die Bedeutung [$N(CH_3)$, 4—$OCH_3$], [$N(CH_3)$, 4—Cl] oder [$CH_2$, H] hat;
$R_1$ eine Alkylgruppe, die bis zu 6 Kohlenstoffatome enthält, bedeutet; und

$R_3$ Wasserstoff oder eine Alkylgruppe, die bis zu 6 Kohlenstoffatome enthält, bedeutet.

11. Verbindung nach Anspruch 10, wobei $R_1$ Ethyl bedeutet.

12. Verbindung nach Anspruch 11, wobei $R_3$ Wasserstoff bedeutet.

13. Verbindung der Formel:

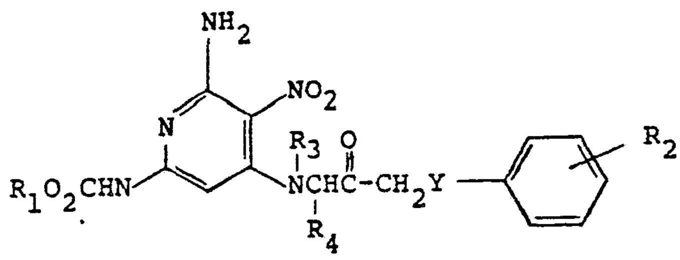

worin

das Paar [Y, $R_2$] die Bedeutung [N($CH_3$), 4—$OCH_3$], [N($CH_3$, 4—Cl] oder [$CH_2$, H] hat;
$R_1$ eine Alkylgruppe, die bis zu 6 Kohlenstoffatome enthält, bedeutet; und
$R_3$ und $R_4$ entweder beide Wasserstoff bedeuten oder eines Wasserstoff und das andere eine Alkylgruppe, die bis zu 6 Kohlenstoffatome enthält, bedeutet.

14. Verbindung nach Anspruch 13, wobei $R_1$ Ethyl bedeutet.

15. Verbindung nach Anspruch 14, wobei $R_3$ und $R_4$ beide Wasserstoff bedeuten.

16. Verfahren zur Herstellung eines 1,2-Dihydropyrido[3,4-b]pyrazins nach Anspruch 1, umfassend das Aminieren eines bis zu 6 Kohlenstoffatome enthaltenden Alkylesters von 6-Amino-4-chlor-5-nitro-pyrimidin-2-ylcarbamat mit dem Oxim eines α-Aminoketons der Struktur:

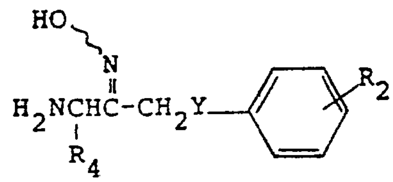

um einen bis zu 6 Kohlenstoffatome enthaltenden Alkylester eines 6-Amino-5-nitro-4-[(2-oxoethyl)amino]pyridin-2-ylcarbamat-oxims der Struktur:

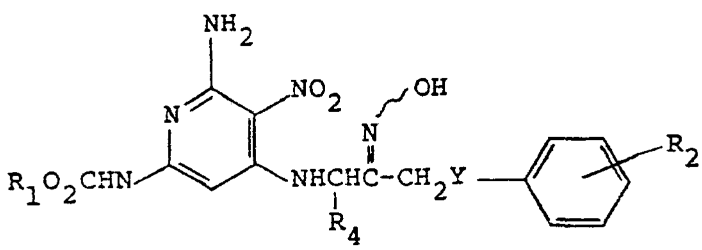

zu erhalten, Hydrolisieren der Verbindung, um das korrespondierende Keton der Struktur:

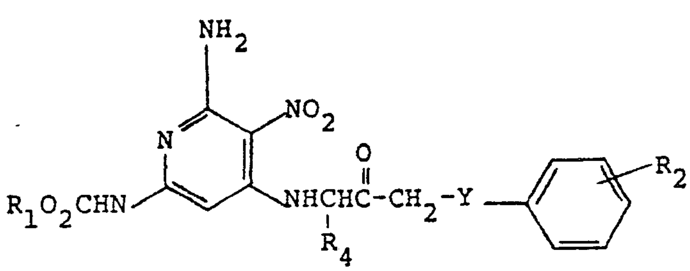

zu erhalten und Unterziehen des Ketons der katalytischen Hydrierung, wobei $R_1$, $R_2$, $R_4$ und Y die in Anspruch 1 definierten Bedeutungen besitzen.

17. Verfahren zur Herstellung eines 1,2-Dihydro-pyrido[3,4-b]pyrazins nach Anspruch 1, umfassend das Aminieren eines bis zu 6 Kohlenstoffatome enthaltenden Alkylesters von 6-Amino-4-chlor-5-nitro-pyrimidin-2-ylcarbamat mit dem Oxim eines α-Aminoketons der Struktur:

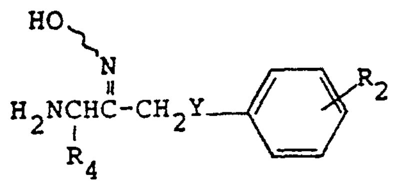

um einen bis zu 6 Kohlenstoffatome enthaltenden Alkylester eines 6-Amino-5-nitro-4-[(2-oxoethyl)amino]pyridin-2-ylcarbamat-oxims der Struktur:

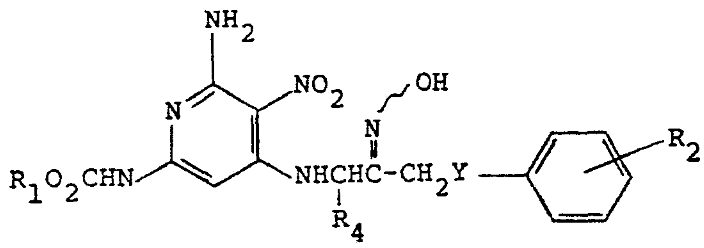

zu erhalten, und Unterziehen der Verbindung der katalytischen Hydrierung, wobei $R_1$, $R_2$, $R_4$ und Y die in Anspruch 1 definierten Bedeutungen besitzen.

18. Verfahren zur Herstellung eines 1,2-Dihydropyrido[3,4-b]pyrazins nach Anspruch 1, umfassend das Aminieren eines bis zu 6 Kohlenstoffatome enthaltenden Alkylesters von 6-Amino-4-chlor-5-nitropyridin-2-ylcarbamat mit einem α-Aminoalkohol der Struktur:

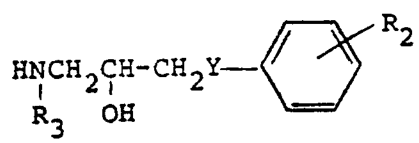

um eine Verbindung der Struktur:

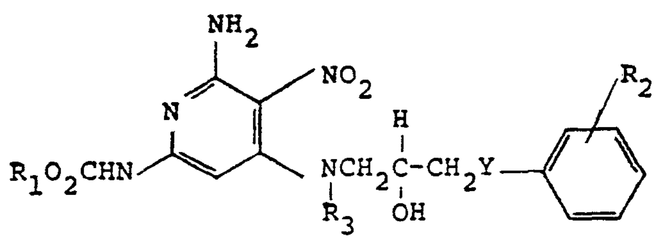

zu erhalten, Oxidieren der Verbindung, um ein Keton der Struktur:

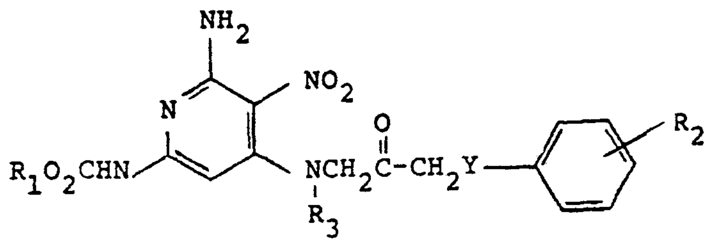

VA

zu erhalten und Unterziehen des Ketons der katalytischen Hydrierung, wobei $R_1$, $R_2$, $R_3$ und Y die in Anspruch 1 definierten Bedeutungen besitzen.

19. Arzneimittel in Form einer Dosiseinheit, umfassend eine wirksame Menge einer Verbindung nach Anspruch 1 in Assoziation mit einem pharmazeutischen Träger.

**Patentansprüche für den Vertragsstaat: AT**

1. Verfahren zur Herstellung eines 1,2-Dihydropyrido[3,4-b]pyrazin der Formel:

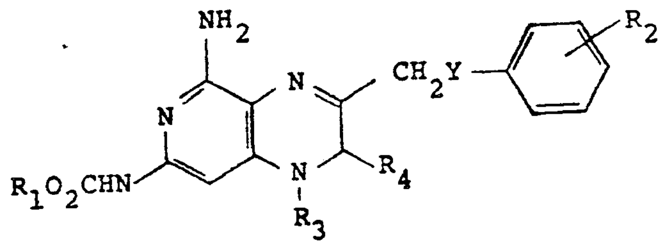

worin
das Paar [Y, $R_2$] die Bedeutung [$N(CH_3)$, 4—$OCH_3$], [$N(CH_3)$, 4—Cl] oder [$CH_2$, H] hat;
$R_1$ eine Alkylgruppe, die bis zu 6 Kohlenstoffatome enthält, bedeutet; und
$R_3$ und $R_4$ entweder beide Wasserstoff bedeuten oder eines Wasserstoff und das andere eine Alkylgruppe, die bis zu 6 Kohlenstoffatome enthält, bedeutet, umfassend das Aminieren eines bis zu 6 Kohlenstoffatome enthaltenden Alkylesters von 6-Amino-4-chlor-5-nitropyrimidin-2-ylcarbamat mit dem Oxim eines α-Aminoketons der Struktur:

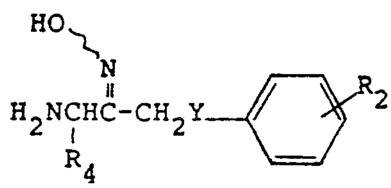

un einen bis zu 6 Kohlenstoffatome enthaltenden Alkylester eines 6-Amino-5-nitro-4-[(2-oxoethyl)amino]pyridin-2-ylcarbamat-oxims der Struktur:

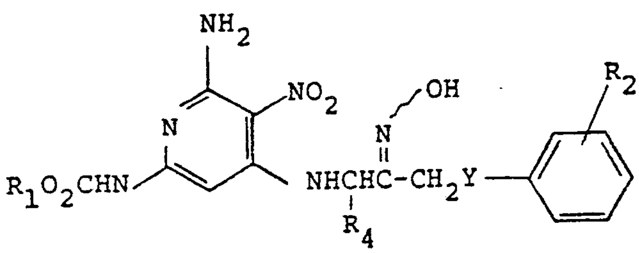

zu erhalten, Hydrolisieren der Verbindung, um das korrespondierende Keton der Struktur:

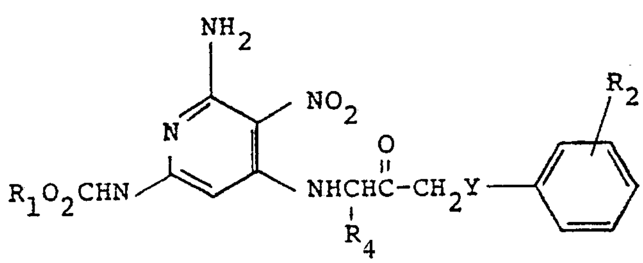

zu erhalten und Unterziehen des Ketons der katalytischen Hydrierung, wobei $R_1$, $R_2$, $R_4$ und Y wie oben definiert sind.

2. Verfahren zur Herstellung eines 1,2-Dihydro-pyrido[3,4-b]pyrazins nach Anspruch 1, umfassend das Aminieren eines bis zu 6 Kohlenstoffatome enthaltenden Alkylesters von 6-Amino-4-chlor-5-nitropyrimidin-2-ylcarbamat mit dem Oxim eines α-Aminoketons der Struktur:

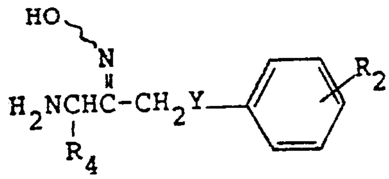

III

um einen bis zu 6 Kohlenstoffatome enthaltenden Alkylester eines 6-Amino-5-nitro-4-[(2-oxoethyl)amino]pyridin-2-ylcarbamat-oxims der Struktur:

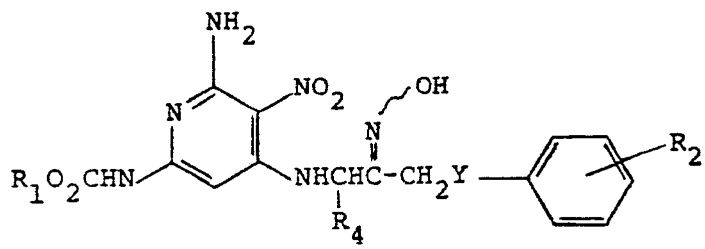

zu erhalten und Unterziehen der Verbindung der katalytischen Hydrierung, wobei $R_1$, $R_2$, $R_4$ und Y die in Anspruch 1 definierten Bedeutungen besitzen.

3. Verfahren zur Herstellung eines 1,2-Dihydropyrido[3,4-b]pyrazins nach Anspruch 1, umfassend das Aminieren eines bis zu 6 Kohlenstoffatome enthaltenden Alkylesters von 6-Amino-4-chlor-5-nitropyridin-2-ylcarbamat mit einem α-Aminoalkohol der Struktur:

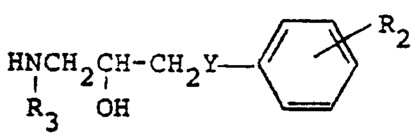

um eine Verbindung der Struktur:

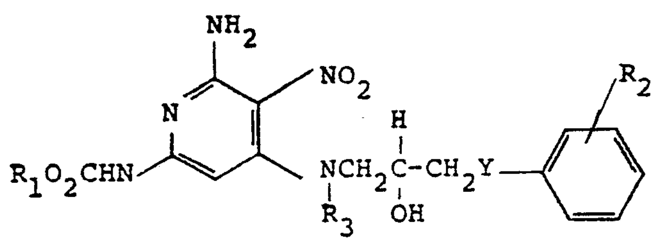

zu erhalten, Oxidieren der Verbindung, um ein Keton der Struktur:

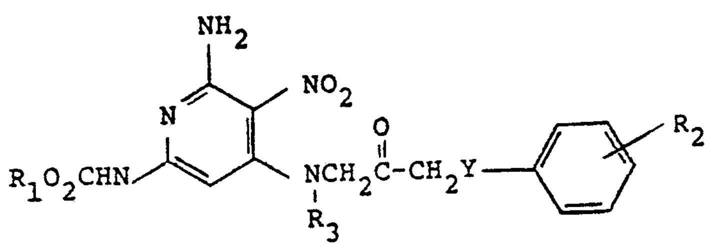

VA

zu erhalten und Unterziehen des Ketons der katalytischen Hydrierung, wobei $R_1$, $R_2$, $R_3$ und Y die in Anspruch 1 definierten Bedeutungen besitzen.

**Revendications pour les Etats Contractants: BE CH DE FR GB IT LI LU NL SE**

1. 1,2-dihydropyrido[3,4-*b*]pyrazine, représentée par la formule:

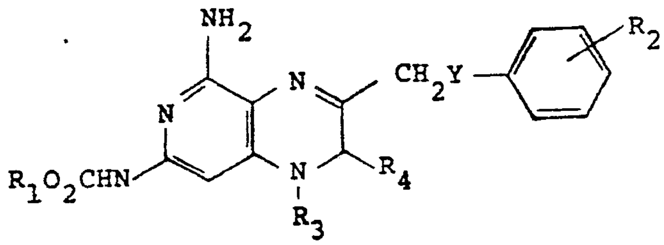

formule dans laquelle:
—la paire [Y, $R_2$] représente [$N(CH_3)$, 4—$OCH_3$], [$N(CH_3)$, 4—Cl] ou [$CH_2$, H];
—$R_1$ représente un groupe alkyle renfermant jusqu'à 6 atomes de carbone; et,
—$R_3$ et $R_4$ représentent, soit tous les deux un atome d'hydrogène, soit l'un, un atome d'hydrogène, et l'autre, un groupe alkyle renfermant jusqu'à 6 atomes de carbone,
et leurs sels pharmaceutiquement acceptables.

2. Composé selon la revendication 1, dans lequel $R_1$ représente un reste éthyle.

3. Composé selon la revendication 2, dans lequel $R_3$ et $R_4$ représentent chacun un atome d'hydrogène.

4. 5-amino-1,2-dihydro-3-[[*N*-(4-méthoxy-phényl)-*N*-méthyl]aminométhyl]pyrido[3,4-*b*]pyrazine-7-carbamate d'éthyle.

5. 5-amino-1,2-dihydro-3-[[*N*-(4-chloro-phényl)-*N*-méthyl]aminométhyl]pyrido[3,4-*b*]pyrazine-7-carbamate d'éthyle.

6. 5-amino-1,2-dihydro-3-(2-phényl-éthyl)pyrido[3,4-*b*]pyrazine-7-carbamate d'éthyle.

7. Composé représenté par la formule:

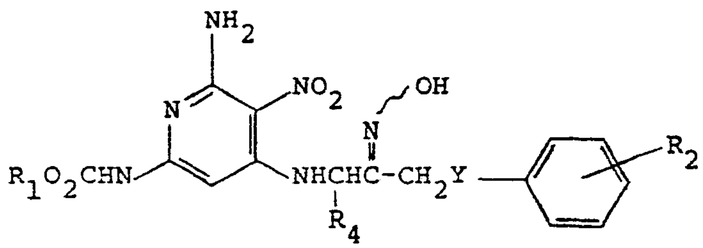

formule dans laquelle:
—la paire [Y, $R_2$] représente [$N(CH_3)$, 4—$OCH_3$], [$N(CH_3)$, 4—Cl] ou [$CH_2$, H];
—$R_1$ représente un groupe alkyle renfermant jusqu'à 6 atomes de carbone; et,
—$R_4$ représente un atome d'hydrogène ou un groupe alkyle renfermant jusqu'à 6 atomes de carbone.

8. Composé selon la revendication 7, dans lequel $R_1$ représente un reste éthyle.

9. Composé selon la revendication 8, dans lequel $R_4$ représente un atome d'hydrogène.

10. Composé représenté par la formule;

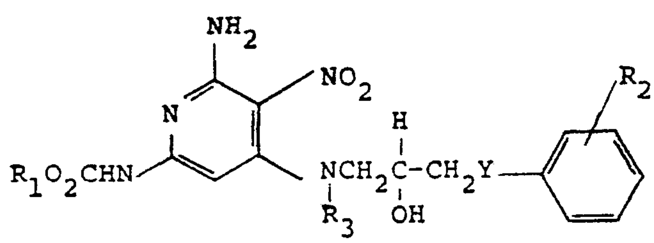

formule dans laquelle:
—la paire [Y, $R_2$] représente [$N(CH_3)$, 4—$OCH_3$], [$N(CH_3)$, 4—Cl] ou [$CH_2$, H];
—$R_1$ représente un groupe alkyle renfermant jusqu'à 6 atomes de carbone; et,
—$R_3$ représente un atome d'hydrogène ou un groupe alkyle renfermant jusqu'à 6 atomes de carbone.

11. Composé selon la revendication 10, dans lequel $R_1$ représente un reste éthyle.

12. Composé selon la revendication 11, dans lequel $R_3$ représente un atome d'hydrogène.

13. Composé représenté par la formule:

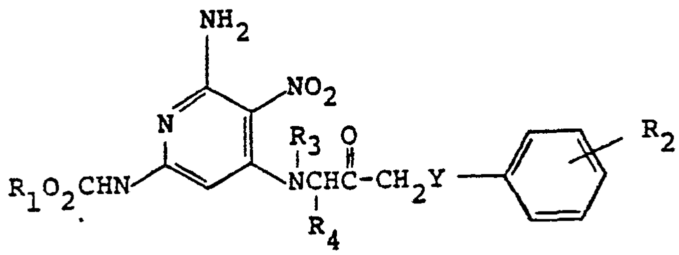

formule dans laquelle:

—la paire [Y, $R_2$] représente [$N(CH_3)$, 4—$OCH_3$], [$N(CH_3)$, 4—Cl] ou [$CH_2$, H];

—$R_1$ représente un groupe alkyle renfermant jusqu'à 6 atomes de carbone; et,

—$R_3$ et $R_4$ représente, soit tous les deux un atome d'hydrogène, soit l'un, un atome d'hydrogène, et l'autre, un groupe alkyle renfermant jusqu'à 6 atomes de carbone.

14. Composé selon la revendication 13, dans laquelle $R_1$ représente un reste éthyle.

15. Composé selon la revendication 14, dans lequel $R_3$ et $R_4$ représentent chacun un atome d'hydrogène.

16. Procédé de préparation d'un 1,2-dihydropyrido[3,4-*b*]pyrazine telle que définie à la revendication 1, qui consiste à

—aminer un alkyl ester de 6-amino-4-chloro-5-nitropyrimidin-2-ylcarbamate, le reste alkyle de l'ester renfermant jusqu'à 6 atomes de carbone, avec l'oxime d'une alpha-amino cétone de structure:

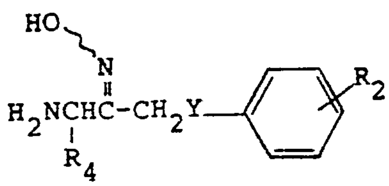

pour obtenir un alkyl ester d'une 6-amino-5-nitro-4-[(2-oxoéthyl) amino]pyridin-2-ylcarbamate oxime, de structure:

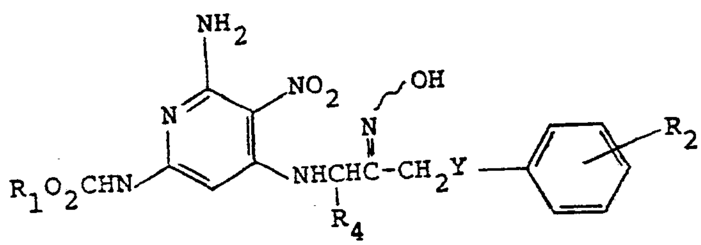

le reste alkyle de l'ester renfermant jusqu'à 6 atomes de carbone,

—hydrolyser ledit composé pour obtenir la cétone correspondante de structure:

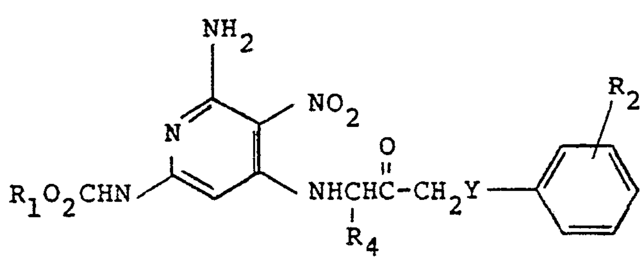

V

et,

—soumettre ladite cétone à une hydrogénation catalytique,

où $R_1$, $R_2$, $R_4$ et Y sont tels que définis à la revendication 1.

17. Procédé de préparation d'une 1,2-dihydropyrido[3,4-*b*]pyrazine telle que définie à la revendication 1, qui consiste à
—aminer un alkyl ester de 6-amino-4-chloro-5-nitropyrimidin-2-ylcarbamate, le reste alkyle de l'ester renfermant jusqu'à 6 atomes de carbone, avec l'oxime d'une alpha-amino cétone de structure:

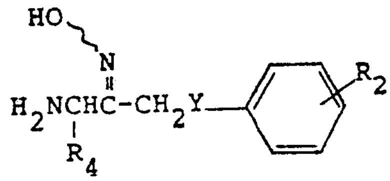

pour obtenir un alkyl ester d'une 6-amino-5-nitro-4-[(2-oxoéthyl)amino]pyridin-2-ylcarbamate oxime, de structure:

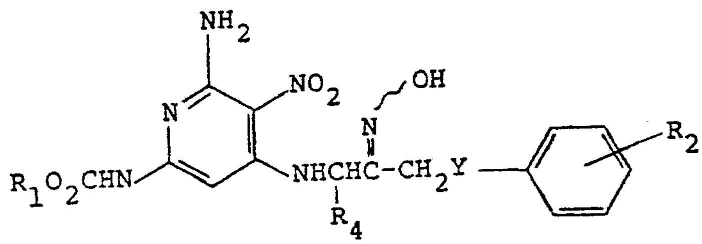

le reste alkyle de l'ester renfermant jusqu'à 6 atomes de carbone,
et,
—soumettre ledit comosé à une hydrogénation catalytique,
où $R_1$, $R_2$, $R_4$ et Y sont tels que définis à la revendication 1.

18. Procédé de préparation d'une 1,2-dihydropyrido[3,4-*b*]pyrazine telle que définie à la revendication 1, qui consiste à aminer un alkyl ester de 6-amino-4-chloro-5-nitropyrimidin-2-ylcarbamate, le reste alkyle de l'ester renfermant jusqu'à 6 atomes de carbone, avec un alpha-amino alcool de structure:

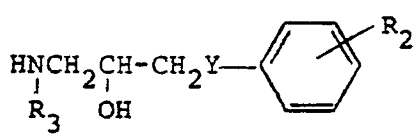

pour donner un composé de structure:

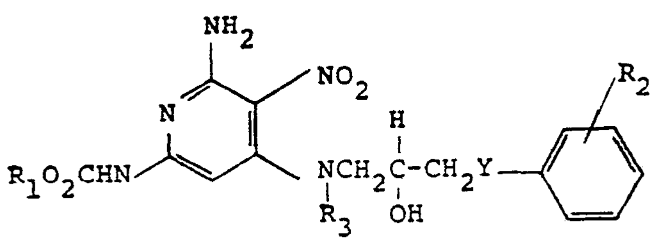

—oxyder ledit composé pour donner une cétone de structure:

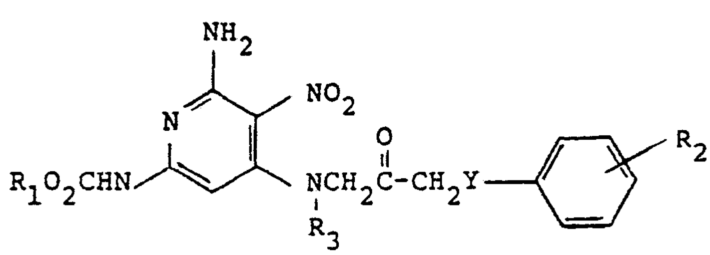

VA

et,
—soumettre ladite cétone à une hydrogénation catalytique,
où $R_1$, $R_2$, $R_4$ et Y sont tels qu définis à la revendication 1.

19. Composition pharmaceutique sous forme d'unités de dosage comprenant une quantité efficace d'un composé tel que défini à la revendication 1 en association avec un support pharmaceutique.

**Revendications pour l'Etat Contractant: AT**

1. Procédé de préparation d'une 1,2-dihydropyrido[3,4-*b*]pyrazine, représentée par la formule:

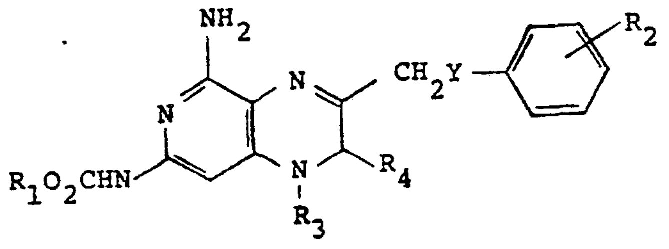

formule dans laquelle:
—la paire [Y, $R_2$] représente [$N(CH_3)$, 4—$OCH_3$], [$N(CH_3)$, 4—Cl] ou [$CH_2$, H];
—$R_1$ représente un groupe alkyle renfermant jusqu'à 6 atomes de carbone; et,
—$R_3$ et $R_4$ représentent, soit tous les deux un atome d'hydrogène, soit l'un, un atome d'hydrogène, et l'autre, un groupe alkyle renfermant jusqu'à 6 atomes de carbone,
qui consiste à
—aminer un alkyl ester de 6-amino-4-chloro-5-nitropyrimidin-2-ylcarbamate, le reste alkyle de l'ester renfermant jusqu'à 6 atomes de carbone, avec l'oxime d'une alpha-amino cétone de structure:

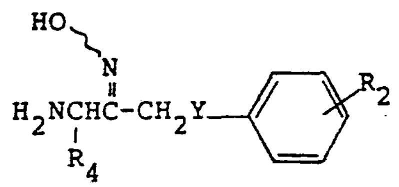

pour obtenir un alkyl ester d'une 6-amino-5-nitro-4-[(2-oxoéthyl) amino]pyridin-2-ylcarbamate oxime, de structure:

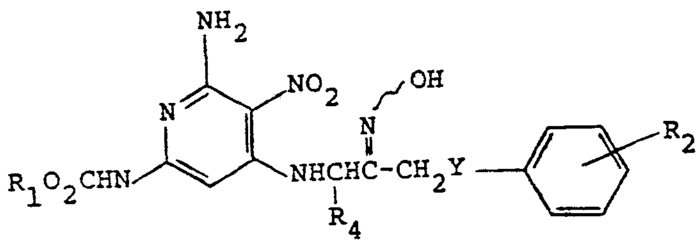

le reste alkyle de l'ester renfermant jusqu'à 6 atomes de carbone,
—hydrolyser ledit composé pour obtenir la cétone correspondante de structure:

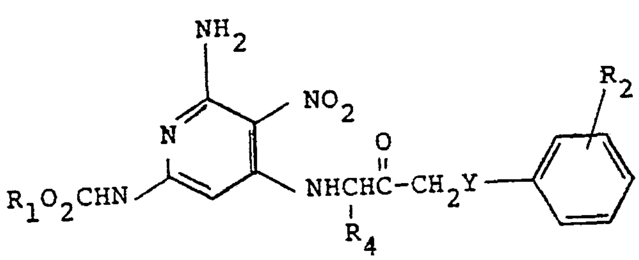

et,
—soumettre ladite cétone à une hydrogénation catalytique,
où $R_1$, $R_2$, $R_4$ et Y sont tels que définis.

2. Procédé de préparation d'une 1,2-dihydropyrido[3,4-*b*]pyrazine telle que définie à la revendication 1, qui consiste à

—aminer un alkyl ester de 6-amino-4-chloro-5-nitropyrimidin-2-ylcarbamate, le reste alkyle de l'ester renfermant jusqu'à 6 atomes de carbone, avec l'oxime d'une alpha-amino cétone de structure:

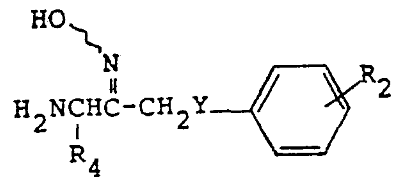

pour obtenir un alkyl ester d'une 6-amino-5-nitro-4-[(2-oxoéthyl)amino]pyridin-2-ylcarbamate oxime, de structure:

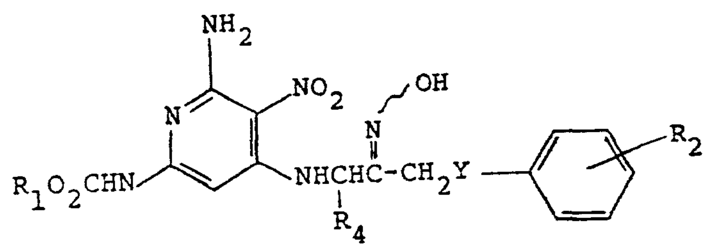

le reste alkyle de l'ester renfermant jusqu'à 6 atomes de carbone,

et,

—soumettre ledit comosé à une hydrogénation catalytique,

où $R_1$, $R_2$, $R_4$ et Y sont tels que définis à la revendication 1.

3. Procédé de préparation d'une 1,2-dihydropyrido[3,4-*b*]pyrazine telle que définie à la revendication 1, qui consiste à

— aminer un alkyl ester de 6-amino-4-chloro-5-nitropyrimidin-2-ylcarbamate, le reste alkyle de l'ester renfermant jusqu' à 6 atomes de carbone, avec un alpha-amino alcool de structure:

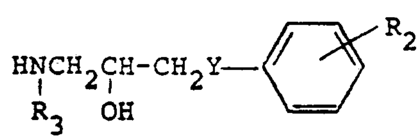

pour donner un composé de structure:

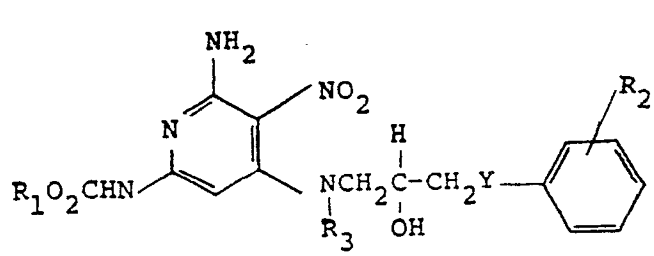

—oxyder ledit composé pour donner une cétone de structure:

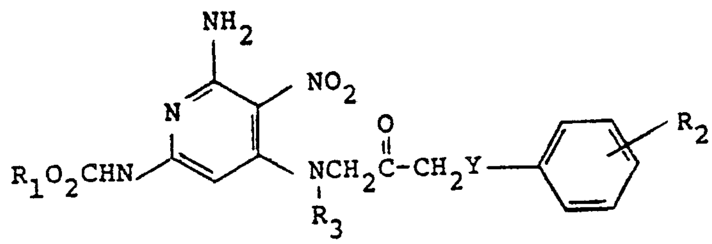

et,
—soumettre ladite cétone à une hydrogénation catalytique, où $R_1$, $R_2$, $R_4$ et Y sont tels que définis à la revendication 1.